(19) Europäisches Patentamt
European Patent Office
Office européen des brevets

(11) Veröffentlichungsnummer: **0 003 060**
**B1**

(12) # EUROPÄISCHE PATENTSCHRIFT

(45) Veröffentlichungstag der Patentschrift: **25.11.81**

(51) Int. Cl.³: **C 07 C 61/16,** C 07 C 51/42 //C07C 87/28

(21) Anmeldenummer: **78101875.9**

(22) Anmeldetag: **29.12.78**

(54) **Verfahren zur Trennung von Stereoisomeren von substituierten Vinylcyclopropancarbonsäuren.**

(30) Priorität: **10.01.78 DE 2800922**

(43) Veröffentlichungstag der Anmeldung: **25.07.79 Patentblatt 79/15**

(45) Bekanntmachung des Hinweises auf die Patenterteilung: **25.11.81 Patentblatt 81/47**

(84) Benannte Vertragsstaaten: **BE CH DE FR GB IT NL**

(56) Entgegenhaltungen: **DE - A - 2 300 325** **DE - A - 2 723 383**

(73) Patentinhaber: **BAYER AG**
**Zentralbereich Patente, Marken und Lizenzen**
**D-5090 Leverkusen 1, Bayerwerk (DE)**

(72) Erfinder: **Naumann, Klaus, Dr.**
**Wolfskaul 2**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 060 B1

Verfahren zur Trennung von Stereoisomeren von substituierten Vinylcyclopropancarbonsäuren

Die vorliegende Erfindung betrifft ein neues Verfahren zur Trennung von bekannten substituierten Vinylcyclopropancarbonsäuren in ihre Stereoisomeren.

Es ist bereits bekannt geworden, daß man Gemische von stereoisomeren cis- bzw. trans-substituierten Cyclopropancarbonsäuren durch fraktionierte Kristallisation trennen kann (Coll. Czech. Chem. Commun. 24, 2230 (1959), Pestic. Sci. 1971, 245; Pestic. Sci. 1974, 791; DOS 2 439 177); Dieses Verfahren ist jedoch mühsam und verlustreich. Zur Gewinnung größerer Mengen reiner trans- oder cis-Isomeren ist dieses Verfahren nicht geeignet.

Es ist außerdem bereits bekannt, daß optisch aktive Säuren aufgrund der unterschiedlichen Löslichkeit ihrer Salze mit Aminen getrennt werden können. Es war jedoch nicht bekannt, daß auf diese Weise eine Trennung der cis-trans Isomeren von Cyclopropancarbonsäuren durchgeführt werden kann. So war aus DE—OS 2 300 325 die Abtrennung von + trans Chrysanthemumsäure aus ihrem (±) cis/trans Gemisch mit Hilfe von optisch aktiven (—)-$\alpha$-(1-Naphthyl)äthylamin bekannt. DE—OS 2 723 383 beschreibt die Razemisierung optisch aktiver Cyclopropancarbonsäurehalogenide. Keine dieser Literaturstellen beschreibt jedoch ein technisch geeignetes Verfahren zur Trennung des cis/trans Gemisches von substituierten Vinylcyclopropancarbonsäuren.

Es wurde nun gefunden, daß man die Stereoisomeren von substituierten Vinylcyclopropancarbonsäuren der allgemeinen Formel (I)

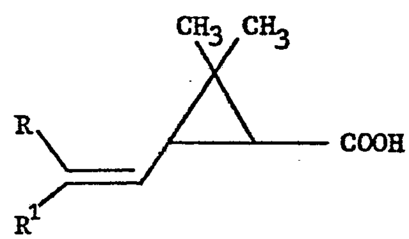

(I)

in welcher

R für Alkyl mit 1 bis 4 C—Atomen, gegebenenfalls durch Alkyl oder Halogen substituierter Phenyl und/oder Halogen steht und

R¹ für Wasserstoff, Alkyl mit 1 bis 4 C—Atomen oder Halogen steht, wobei R und R¹ auch gemeinsam mit den angrenzenden C—Atom einen carbocyclischen Ring bilden können.

Die Stellung der Substituenten am Ring ist cis- oder trans. Die Moleküle können auch in der optisch aktiven Form vorliegen.

auf einfach Weise trennen kann, indem man die freien Vinylcyclopropancarbonsäuren in schwach alkalischer, wäßriger Lösung mit einer dem cis-Anteil entsprechenden Menge an Aminsalz umsetzt, die entsprechenden cis/trans-isomeren Salze aufgrund ihrer unterschiedlichen Löslichkeit voneinander trennt, und die cis- bzw. trans-isomeren Vinylcyclopropancarbonsäuren mit Hilfe von starken Säuren in Freiheit setzt und umkristallisiert.

Es ist als ausgesprochen überraschend zu bezeichnen, daß gemäß dem erfindungsgemäßen Verfahren die Trennung der cis-isomeren-Säure von der trans-isomeren-Säure gelingt, da die so stark unterschiedliche Löslichkeit der cis/trans-isomeren Salze nicht vorhersehbar war.

Weiterhin ist überraschend, daß die Trennung dann besonders gut verläuft, wenn man eine dem jeweiligen cis-Anteil entsprechende Menge an Aminsalz einsetzt.

Das erfindungsgemäße Verfahren weist eine Reihe von Vorteilen auf. So lassen sich die gelegentlich in nur untergeordneten Mengen in einem cis-trans-Gemisch vorhandenen cis-Isomeren von der Hauptmenge der anderen Isomeren abtrennen, was sowohl zur Reinigung als auch zu Anreicherungszwecken dienen kann. Weiter ist das erfindungsgemäße Verfahren auch zur Gewinnung größerer Mengen der jeweiligen Isomeren geeignet und kann kontinuierlich betrieben werden.

Verwendet man z.B. 2 Mol 2,2-Dichlorvinyl-3,3-dimethyl-cyclopropancarbonsäure in Form des 1:1 cis/trans-Gemisches, wäßrige Natriumcarbonat-Lösung als alkalisches Medium, 1 Mol racemisches $\alpha$-Methylbenzylamin-hydrochlorid als Aminsalz und Salzsäure als freisetzende Säure, so kann der Reaktionsablauf durch das folgende Formelschema wiedergegeben werden:

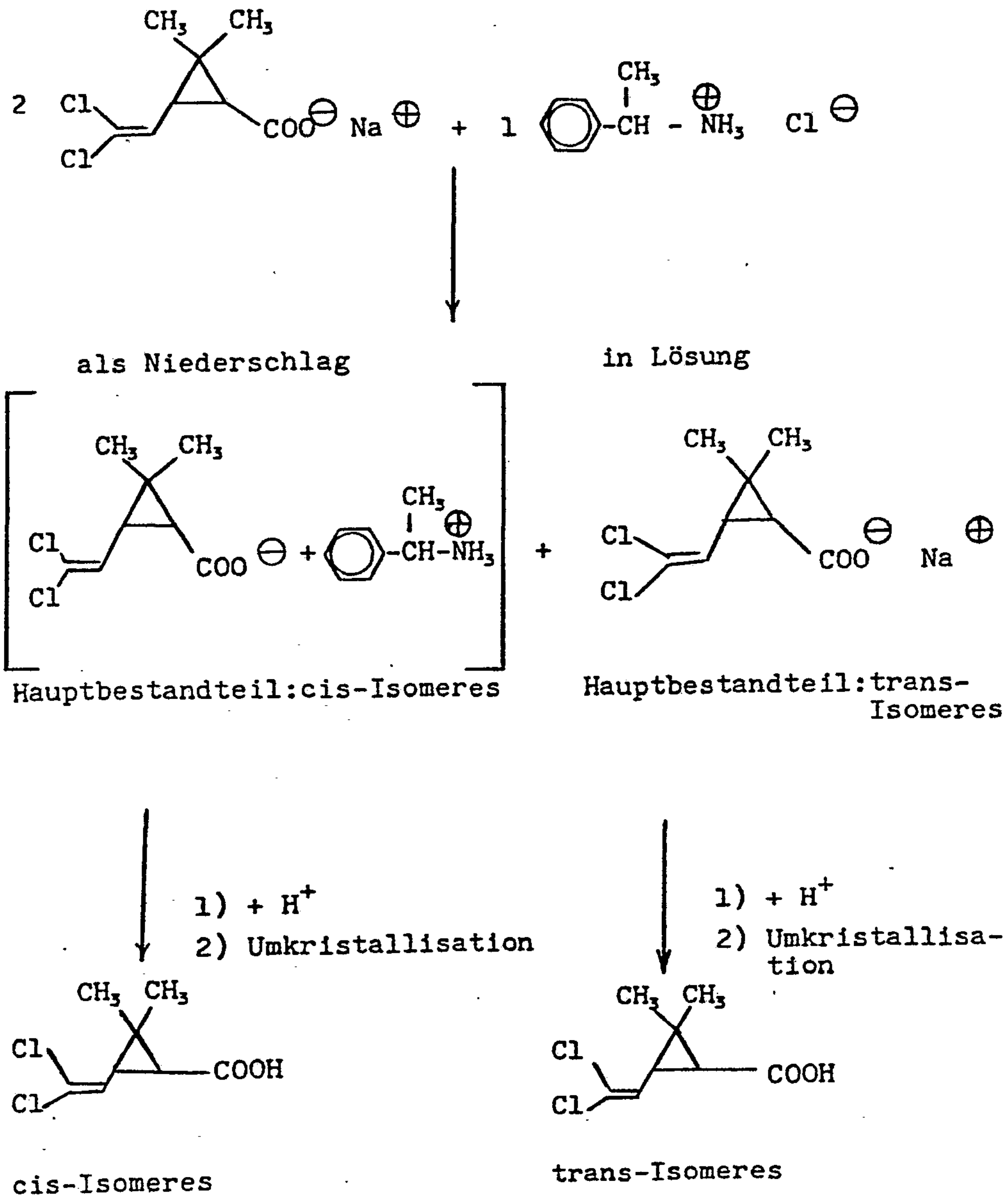


Durch die Formel (I) sind die durch das erfindungsgemäße Verfahren in ihre Isomeren trennbaren substituierten Vinylcyclopropancarbonsäuren allgemein definiert. Vorzugsweise steht R für Methyl, Chlor und/oder Brom.

Als beispiel für die nach dem erfindungsgemäßen Verfahren in die Stereoisomeren trennbaren Verbindungen seien im Einzelnen aufgeführt:

2-(2,2-Dichlorvinyl)-3,3-dimethyl-cyclopropancarbonsäure
2-(2,2-Dibromvinyl)-3,3-dimethyl-cyclopropancarbonsäure
2-(2-Methylbuten-1-yl)-3,3-dimethyl-cyclopropancarbonsäure

Die als Ausgangsmaterial verwendeten Isomerengemische von substituierten Vinylcyclopropancarbonsäuren der allgemeinen Formel (I) sind bekannt (Coll. Czech. Chem. Commun. 24, 2230 (1959); DOS 2 615 159; J. Agr. Food Chem. 21, 767 (1973); Brit. Pat. 1 446 304; Pestic. Sci. 1971, 245; Pestic. Sci. 1974, 537; Brit. Pat. 1 413 491; J. Chem. Soc. 1945, 285; DOS 2 432 951; J. Org. Chem. 17, 381 (1952), DOS 2 439 177).

Für das erfindungsgemäße Verfahren kommen als Aminsalze vorzugsweise Salze von Arylalkylaminen in Frage. Hierzu seien beispielsweise die Hydrochloride von Benzylamin, $\alpha$-Methylbenzylamin, Phenäthylamin und Amino-phenylessigsäureäthylester genannt.

Das erfindungsgemäße Verfahren wird in Gegenwart von Puffersubstanzen, welche ein konstantes, schwach alkalisches Milieu aufrechterhalten, durchgeführt. Dafür werden vorzugsweise wässrige Lösungen anorganischer Salze mehrbasiger Säuren, wie z.B. der Phosphor- oder Kohlensäure verwendet. Hierzu gehören vorzugsweise Natriumcarbonat, Kaliumcarbonat, Natriumhydrogencarbonat oder Natriumhydrogenphosphat.

Die cis- bzw. trans- Cyclopropancarbonsäuren werden nach dem erfindungsgemäßen Verfahren durch Zugabe von starken Säuren aus den entsprechenden Salzen in Freiheit gesetzt. Als Säuren können dabei vorzugsweise anorganische Säuren, wie Salzsäure oder Schwefelsäure, oder organische Säuren, wie Ameisensäure, Trichloressigsäure und Trifluoressigsäure, verwendet werden.

Bei der Durchführung des erfindungsgemäßen Verfahrens verwendet man pro Mol Isomerengemisch von Vinylcyclopropancarbonsäure eine dem cis-Anteil entsprechende Menge Aminsalz sowie molare Mengen der Puffersubstanz. Die Reaktionspartner können bei Raumtemperatur oder auch in der Hitze zusammengegeben werden und man läßt dann langsam auskristallisieren. Während das Aminsalz der Cyclopropancarbonsäure, das überwiegend aus cis-Isomeren besteht, als die schwerer lösliche Komponente der ausfallende Teil ist, bleibt das Natriumsalz der Cyclopropancarbonsäure, das überwiegend aus trans-Isomeren besteht, als die leichter lösliche Komponente in Lösung. Aus beiden Salzen werden durch Zugabe der äquivalenten Menge einer starken Säure jeweils die freien, an cis- bzw. trans- stark angereicherten, Cyclopropancarbonsäuren erhalten. Das dabei freiwerdende Aminsalz kann für weitere Fällungen rückgeführt werden. Durch Umkristallisation werden die reine cis- und die reine trans-Form erhalten. Dabei bleibt jeweils ein gewisser Anteil als ein 1:1 Gemisch aus cis- und trans-Isomeren in Lösung, der jedoch wieder in die anfängliche Fällung eingebracht werden kann.

Als Lösungsmittel zum Umkristallisieren eignen sich unpolare Lösungsmittel wie Alkane mit bis zu 8 Kohlenstoffatomen, Petroläther oder Cyclohexan, ferner Halogenalkane wie Tetrachlormethan, Chlorfluormethan, aber auch stark polare Lösungsmittel wie wässriger Alkohol, Ketone oder Aether.

Die nach dem erfindungsgemäßen Verfahren erhaltenen stereoisomeren substituierten Vinylcyclopropancarbonsäuren der Formel (I) werden für die Herstellung von hochwirksamen Insektiziden verwendet.

Das folgende Beispiel illustriert das erfindungsgemäße Verfahren, ohne eine Einschränkung hinsichtlich der Breite seiner Verwendbarkeit anzugeben.

Beispiel 1

1 Mol des Isomerengemisches von 2-(2,2-Dichlorvinyl)-3,3-dimethylcyclopropancarbonsäure (Verhältnis cis/trans = 50/50) wird in 1 Mol Natriumcarbonat in 1,5 l Wasser gelöst. Zu der schwach alkalischen Lösung werden bei 20°C 0,5 Mol racemisches $\alpha$-Methylbenzylamin-hydrochlorid in 0,5 l Wasser getropft, wobei sich ein Niederschlag bildet.

Der entstandene Niederschlag, das nahezu quantitativ gebildete $\alpha$-Methylbenzylaminsalz der Cyclopropancarbonsäure, wird abgetrennt und mit Äther und der äquivalenten Menge Salzsäure so lange geschüttelt, bis alles Salz gelöst ist. Man trennt die Wasserphase ab, wäscht die organische Phase nochmals mit verdünnter Salzsäure, trocknet über Natriumsulfat und engt ein. Man erhält freie 2-(2,2-Dichlorvinyl)-3,3-dimethylcyclopropancarbonsäure (Verhältnis cis/trans = 80/20, mit NMR festgestellt).

Aus Petroläther kristallisiert die reine cis-Form aus, während ein 1/1-Gemisch aus cis- und trans-Form noch in Lösung bleibt, das in die Trennung wieder eingebracht werden kann. Die wässrige Phase enthält das wieder freigewordene Aminsalz, das für weitere Fällungen rückgeführt wird.

Aus der wässrigen alkalischen Mutterlauge, die das gelöste Natriumsalz der Cyclopropancarbonsäure enthält, wird in entsprechender Weise die freie 2-(2,2-Dichlorvinyl)-3,3-dimethylcyclopropancarbonsäure (Verhältnis cis/trans = 20/80) erhalten. Aus Petroläther kristallisiert bei 0°C die reine trans-Form aus, während auch hier ein 1/1-Gemisch aus cis-und trans-Form noch in Lösung bleibt, das ebenfalls in die Trennung wieder eingebracht werden kann.

Aus dem eingesetzten 1 Mol des Isomerengemisches von 2-(2,2-Dichlorvinyl)-3,3-dimethylcyclopropancarbonsäure (Verhältnis cis/trans = 50/50) erhält man:

0,3 Mol reine cis-Säure
0,3 Mol reine trans-Säure und
0,4 Mol 1/1-Gemisch aus cis- und trans-säure, das wieder in die Trennung eingebracht werden kann.

**Patentansprüche**

1. Verfahren zur Trennung von Stereoisomeren von substituierten Vinylcyclopropancarbonsäuren der allgemeinen Formel (I)

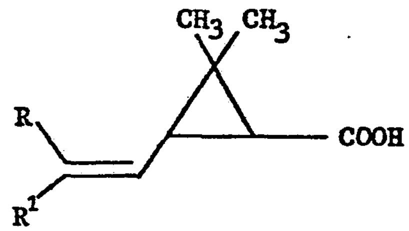

(I)

in welcher

R für Alkyl mit 1—4 C—Atomen, gegebenenfalls durch Alkyl oder Halogen substituiertes Phenyl und/oder Halogen steht und

$R^1$ für Wasserstoff, Alkyl mit 1 bis 4 C—Atomen oder Halogen steht, wobei R und $R^1$ auch gemeinsam mit dem angrenzenden C—Atom einen carbocyclischen Ring bilden können;

dadurch gekennzeichnet, daß man die freien Vinylcyclopropancarbonsäuren in schwach alkalischer, wässriger Lösung mit einer dem cis-Anteil entsprechenden Menge an Aminsalz umsetzt, die entsprechenden cis/trans-isomeren Salze aufgrund ihrer unterschiedlichen Löslichkeit voneinander trennt, und die cis- bzw. trans-isomeren Vinylcyclopropancarbonsäuren mit Hilfe von starken Säuren in Freiheit setzt und umkristallisiert.

2. Verfahren gemäß Anspruch 1, dadurch gekennzeichnet, daß als Aminsalz ein Salz von Arylalkylaminen verwendet wird.

3. Verfahren gemäß Anspruch 1, dadurch gekennzeichnet, daß als Aminsalz ein Salz von $\alpha$-Methylbenzylamin verwendet wird.

4. Verfahren gemäß Anspruch 1, dadurch gekennzeichnet, daß substituierte Vinylcyclopropancarbonsäuren der allgemeinen Formel (I), in welcher R und $R^1$ für Methyl, Chlor oder Brom stehen, eingesetzt werden.

5. Verfahren gemäß Anspruch 1, dadurch gekennzeichnet, daß Permethrinsäure der Formel

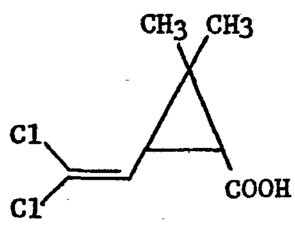

eingesetzt wird.

**Claims**

1. Process for the separation of stereoisomers of substituted vinylcyclopropanecarboxylic acids of the general formula (I)

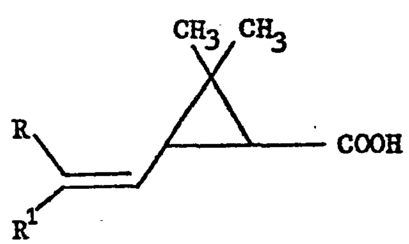

(I)

in which

R represents alkyl with 1 to 4 C atoms, phenyl which is optionally substituted by alkyl or halogen and/or halogen and

$R^1$ represents hydrogen, alkyl with 1 to 4 C atoms or halogen,

and wherein

R and $R^1$, together with the adjacent C atom, can also form a carbocyclic ring;

characterised in that the free vinylcyclopropanecarboxylic acids are reacted with an amount of an

amine salt corresponding to the proportion of cis-isomer in a weakly alkaline, aqueous solution, the corresponding cis-/trans-isomeric salts are separated from one other on the basis of their different solubility and the cis- and trans-isomeric vinylcyclopropanecarboxylic acids are liberated with the aid of strong acids and recrystallised.

2. Process according to Claim 1, characterised in that the amine salt used is a salt of arylalkylamines.

3. Process according to Claim 1, characterised in that the amine salt used is a salt of $\alpha$-methylbenzylamine.

4. Process according to Claim 1, characterised in that substituted vinylcyclopropanecarboxylic acids of the general formula (I), in which R and $R^1$ represent methyl, chlorine or bromine, are used.

5. Process according to Claim 1, characterised in that permethric acid of the formula

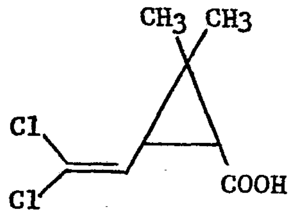

is used.

**Revendications**

1. Procédé pour séparer les stéréomères d'acides vinylcyclopropanecarboxyliques substitués répondant à la formule générale I

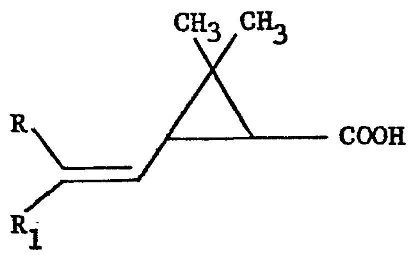

(I)

dans laquelle

R représente un groupe alkyle en C1—C4, un groupe phényle éventuellement susbtitué par des groupes alkyle ou des halogènes, et/ou un halogène, et

$R_1$ représente l'hydrogène, un groupe alkyle en C1—C4 ou un halogène,

R et $R_1$ pouvant également former ensemble et avec l'atome de carbone voisin un noyau carbocyclique,

caractérisé en ce que l'on fait réagir les acides vinylcyclopropanecarboxyliques libres en solution aqueuse légèrement alcaline avec un sel d'amine en quantité correspondant à la proportion de l'isomère cis, on sépare les sels isomères cis/trans correspondants en exploitant leurs solubilités différentes, on libère les acides vinylcyclopropanecarboxyliques isomères cis et trans à l'aide d'acides forts et on les recristallise.

2. Procédé selon la revendication 1, caractérisé en ce que le sel d'amine utilisé est un sel d'arylalkylamine.

3. Procédé selon la revendication 1, caractérisé en ce que le sel d'amine utilisé est un sel de l'$\alpha$-méthylbenzylamine.

4. Procédé selon la revendication 1, caractérisé en ce que l'on met en oeuvre des acides vinylcyclopropanecarboxyliques substitués de formule générale I dans laquelle R et $R_1$ représentent des groupes méthyle, des atomes de chlore ou de brome.

5. Procédé selon la revendication 1, caractérisé en ce que l'on met en oeuvre l'acide perméthrique de formule